# EUROPEAN PATENT APPLICATION

(11) **EP 4 190 775 A1**
(43) Date of publication of application: **07.06.2023**
(21) Application number: 21850254.0
(22) Date of filing: 02.08.2021
(51) Int. Cl.: C07D 403/12, A61K 31/517, A61P 35/00

(54) **CRYSTAL FORM OF FIBROBLAST GROWTH FACTOR RECEPTOR INHIBITOR AND PREPARATION METHOD THEREFOR**

(30) Priority: 31.07.2020 CN 202010756000
(71) Applicant: Transthera Sciences (Nanjing), Inc., Jiangbei New Area Nanjing Jiangsu 210032 (CN)
(72) Inventor: WU, Frank, Nanjing, Jiangsu 210032 (CN)
(74) Representative: Grünecker Patent- und Rechtsanwälte PartG mbB
(86) International application number: PCT/CN2021/110008
(87) International publication number: WO 2022/022735

(57) **Abstract**

The present invention belongs to the technical field of medicine, and relates in particular to a crystal form of a fibroblast growth factor receptor inhibitor shown in formula (I), and a preparation method therefor.

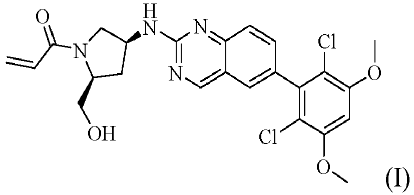

## Description

### TECHNICAL FIELD

The present invention relates to the technical field of pharmaceutics, and in particular to a crystal form of a fibroblast growth factor receptor inhibitor and a method for preparing the same.

### BACKGROUND

Fibroblast growth factor receptors (FGFRs) are important members of the tyrosine kinase receptor family and include 4 types, FGFR-1, FGFR-2, FGFR-3 and FGFR-4. Most of them are single-chain glycoprotein molecules with a molecular weight of 110-150 kD and a structure composed of an extracellular region, a transmembrane region and an intracellular region. Under normal physiological conditions, FGFR binds to its ligand fibroblast growth factor (FGF), and then undergoes dimerization and self-phosphorylation, thereby activating downstream signaling pathways such as JAK/STAT pathway, phospholipase C pathway, phosphoinositide-3-kinase (PI3K) and MAPK signaling pathways, which play important roles in tumor growth and angiogenesis. The abnormal high expression of FGFR is closely related to the occurrence and development of various tumors, such as lung cancer, liver cancer, brain glioma, rhabdomyosarcoma and melanoma.

The compound 1-((2*S*,4*S*)-4-((6-(2,6-dichloro-3,5-dimethoxyphenyl)quinazolin-2-yl)amino)-2-(hydroxymethyl)pyrrolidin-1-yl)prop-2-en-1-one, of the following formula (I), is an inhibitor of FGFR, of which a preparation method is described in WO2018040885A1 giving a pale yellow solid amorphous form with an XRPD pattern shown in FIG. 2. This compound has better inhibitory activity on FGFR and has clinical application potential in treating or preventing related diseases mediated by FGF/FGFR.

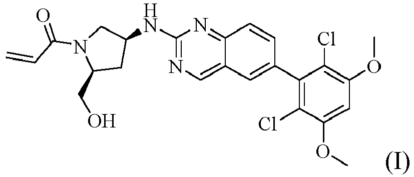

In the R&D process of drugs, studies on crystal forms are very important. Compared with other forms, the crystal forms of compounds are very different in terms of stability, related substances and the like. The inventor conducted a study on a compound of formula (I) in order to obtain a crystal form of the compound.

### SUMMARY

The present invention is intended to provide a crystal form of a compound of formula (I) and a method for preparing the same.

The present invention provides a crystal form I of the compound of formula (I):
The crystal form I of compound 1-((2*S*,4*S*)-4-((6-(2,6-dichloro-3,5-dimethoxyphenyl)quinazolin-2-yl)amino)-2-(hydroxymethyl)pyrrolidin-1-yl)prop-2-en-1-one of formula (I) comprises characteristic peaks in an X-ray powder diffraction pattern using Cu-Kα radiation at 2θ angles of 5.2±0.2°, 10.5±0.2°, 13.9±0.2°, 16.0±0.2°, 20.9±0.2°, 24.4±0.2° and 26.1±0.2°,

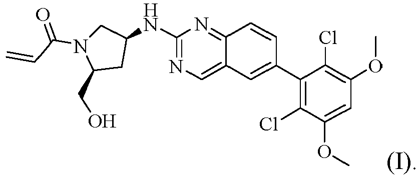

In one embodiment of the present invention, the crystal form I of the compound of formula (I) further comprises characteristic peaks in an X-ray powder diffraction pattern using Cu-Kα radiation at 2θ angles of 11.5±0.2°, 12.9±0.2° and 22.4±0.2° in addition to the characteristic peaks described above.

In one embodiment of the present invention, the crystal form I of the compound of formula (I) further comprises characteristic peaks in an X-ray powder diffraction pattern using Cu-Kα radiation at 2θ angles of 16.4±0.2° and 18.9±0.2° in addition to the characteristic peaks described above.

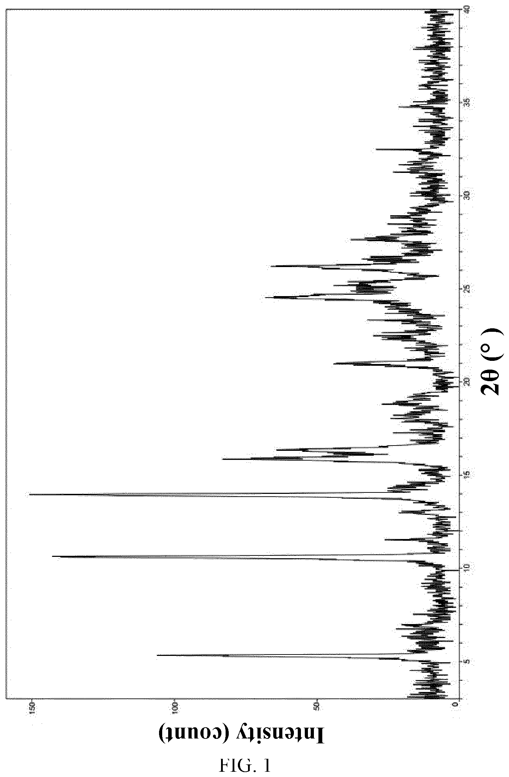

In one embodiment of the present invention, the crystal form I of the compound of formula (I) has an X-ray powder diffraction pattern using Cu-Kα radiation as substantially shown in FIG. 1.

The present invention further provides a method for preparing the crystal form I of the compound of formula (I), comprising:
dissolving a compound of formula (I) in a single or mixed solvent, heating and cooling, and optionally, repeating the process of "heating and cooling" 1-2 times to give the crystal form I.

In one embodiment of the present invention, the single or mixed solvent is selected from one of or a mixture of two or more of methanol, ethanol, isopropanol, toluene, acetone, tetrahydrofuran, dichloromethane, dichloroethane, ethyl acetate, acetonitrile, methyl tert-butyl ether, 2-methyltetrahydrofuran, dimethyl sulfoxide and water; and preferably, the single or mixed solvent is selected from methanol, ethanol, ethyl acetate, acetonitrile, tetrahydrofuran, isopropanol, methyl *tert*-butyl ether, acetone, toluene, dichloromethane, isopropanol/water, and ethanol/water.

In one embodiment of the present invention, the fresh crystal form of the crystal form I does not produce destructive impurities after storage at 105 °C for 3 days, 5 days or 10 days, or after storage at 60 °C for 10 days, 20 days or 30 days.

In one embodiment of the present invention, the mixed solvent is a mixture of water with isopropanol, methanol or ethanol in a volume ratio of 1:(1-5), preferably 1:(1.5-4), and more preferably 1:3.

In one embodiment of the present invention, the heating refers to heating to a temperature of 35 °C or higher, preferably 40 °C to 100 °C.

In one embodiment of the present invention, the cooling refers to cooling to a temperature below 40 °C, preferably room temperature. The room temperature refers to a natural indoor temperature, usually 15 °C to 25 °C.

In one embodiment of the present invention, the single or mixed solvent is used in an amount of 4-40 times the volume of the compound of formula (I).

In one embodiment of the present invention, the single or mixed solvent is selected from methanol, ethanol, ethyl acetate, acetonitrile, tetrahydrofuran, isopropanol, methyl *tert*-butyl ether, acetone, toluene, dichloromethane, isopropanol/water, and ethanol/water; preferably, the solvents and crystallization conditions are as follows: ethanol/water (3/1, 80 °C to 40 °C to 80 °C to room temperature), methanol (cooling from 70 °C to room temperature), ethyl acetate (cooling from 80 °C to room temperature), ethanol (cooling from 90 °C to room temperature), acetonitrile (cooling from 90 °C to room temperature), tetrahydrofuran (cooling from 80 °C to room temperature), isopropanol (cooling from 90 °C to room temperature), isopropanol/water (4:6, cooling from 90 °C to room temperature), methyl tert-butyl ether (cooling from 50 °C to room temperature), acetone (cooling from 70 °C to room temperature), toluene (cooling from 100 °C to room temperature), dichloromethane (cooling from 40 °C to room temperature), and ethanol/water (8:2, cooling from 90 °C to room temperature).

The present invention further provides a pharmaceutical composition comprising the crystal form I of the compound of formula (I) and one or more second therapeutically active agents.

The present invention further provides a pharmaceutical formulation comprising the crystal form I of the compound of formula (I).

In some embodiments of the present invention, the pharmaceutical formulation may comprise one or more pharmaceutically acceptable carriers.

The pharmaceutical carrier described herein may be one or more solid or liquid fillers suitable for administration in human. The pharmaceutical carrier preferably has sufficient purity and sufficiently low toxicity, and is compatible with the compound provided herein without significantly decreasing its efficacy. For example, the pharmaceutical carrier may be a filler, a binder, a disintegrant, a lubricant, an aqueous solvent, a nonaqueous solvent, and the like.

The pharmaceutical formulation described herein may be formulated into any pharmaceutically acceptable dosage form to administer a "therapeutically effective amount" of the aforementioned crystal form I of the compound of formula (I) to a patient or a subject in need of such treatment in any suitable route of administration, such as oral, parenteral, rectal or pulmonary administration. For oral administration, it can be formulated into tablets, capsules, pills, granules, and the like. For parenteral administration, it can be formulated into injections, sterile powders for injection, and the like.

The present invention further provides use of the crystal form I of the compound of formula (I), or the pharmaceutical formulation or the pharmaceutical composition comprising the crystal form I in preparing a medicament for treating or preventing a related disease mediated by FGF/FGFR; preferably, the related disease mediated by FGF/FGFR is a related disease caused by changes in FGFR signaling pathway; specifically, the related disease caused by changes in FGFR signaling pathway is a cancer; the cancer is a solid tumor or a hematological tumor; the cancer includes gallbladder carcinoma, cholangiocarcinoma, lung cancer, squamous cell carcinoma, bladder cancer, gastric cancer, ovarian cancer, peritoneal cancer, breast cancer, breast ductal carcinoma, head and neck cancer, endometrial cancer, corpus carcinoma, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, large intestine villous adenoma, melanoma, cytoma, sarcoma and myelodysplastic syndrome.

The present invention further provides use of the crystal form I of the compound of formula (I) or the pharmaceutical formulation or the pharmaceutical composition comprising the crystal form I in treating or preventing a related disease mediated by FGF/FGFR.

The present invention further provides a method for treating or preventing a related disease mediated by FGF/FGFR, comprising administering to a patient in need a therapeutically effective amount of the aforementioned crystal form I of formula (I), or the pharmaceutical formulation or the pharmaceutical composition comprising the crystal form I.

### DETAILED DESCRIPTION

The "room temperature" described herein refers to a natural indoor temperature, usually 15-25 °C.

The "times the volume" described herein refers to the volume (mL) of solvent required to dissolve 1 g of a substance; for example, if 20 mL of solvent is required to dissolve 1 g of a compound of formula (I), it is referred to as 20 times the volume.

The "therapeutically effective amount" described herein refers to an amount of the aforementioned compound or a pharmaceutically acceptable salt or stereoisomer thereof, the composition or the pharmaceutical formulation thereof that, when administered to a patient, is at least capable of alleviating symptoms of the patient's condition. An actual amount comprising the "therapeutically effective amount" will vary depending on a variety of circumstances, including, but not limited to, the particular condition being treated, the severity of the condition, the physique and health of the patient, and the route of administration. The appropriate amount can be readily determined by skilled medical practitioners using methods known in the medical field.

The "destructive impurity" described herein refers to an additional peak in a liquid chromatogram of a sample in a stability test compared with the liquid chromatogram of the sample on day 0.

In the present invention, the expression "heating and cooling, and optionally, repeating the process of 'heating and cooling' 1-2 times" refers to performing the process of `heating and then cooling' once, or performing the process of `heating and then cooling' 2-3 times.

The present invention also relates to the following technical schemes:
1. A crystal form I of compound 1-((2*S*,4*S*)-4-((6-(2,6-dichloro-3,5-dimethoxyphenyl)quinazolin-2-yl)amino)-2-(hydroxymethyl)pyrrolidin-1 -yl)prop-2-en-1 - one of formula (I), wherein the crystal form I comprises characteristic peaks in an X-ray powder diffraction pattern using Cu-Kα radiation at 2θ angles of 5.2±0.2°, 10.5±0.2°, 13.9±0.2°, 16.0±0.2°, 20.9±0.2°, 24.4±0.2° and 26.1±0.2°, 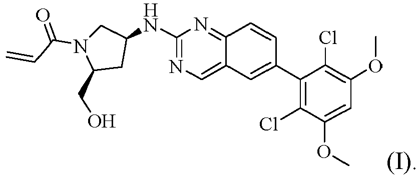
2. The crystal form I according to technical scheme 1, further comprising characteristic peaks in an X-ray powder diffraction pattern using Cu-Kα radiation at 2θ angles of 11.5±0.2°, 12.9±0.2° and 22.4±0.2°.
3. The crystal form I according to technical scheme 2, having an X-ray powder diffraction pattern using Cu-Kα radiation as substantially shown in FIG. 1.
4. A crystal form I of compound 1-((2*S*,4*S*)-4-((6-(2,6-dichloro-3,5-dimethoxyphenyl)quinazolin-2-yl)amino)-2-(hydroxymethyl)pyrrolidin-1 -yl)prop-2-en-1 - one of formula (I), wherein the crystal form I comprises characteristic peaks in an X-ray powder diffraction pattern using Cu-Kα radiation at 2θ angles of 5.2±0.2°, 10.5±0.2°, 13.9±0.2°, 16.0±0.2°, 16.4±0.2°, 24.4±0.2° and 26.1±0.2°, 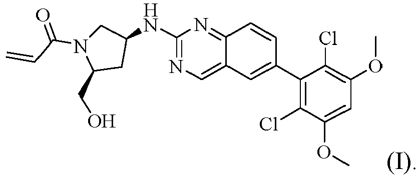
5. The crystal form I according to technical scheme 4, further comprising characteristic peaks in an X-ray powder diffraction pattern using Cu-Kα radiation at 2θ angles of 5.2±0.2°, 10.5±0.2°, 11.5±0.2°, 12.9±0.2°, 13.9±0.2°, 16.0±0.2°, 16.4±0.2°, 18.9±0.2°, 20.9±0.2°, 22.4±0.2°, 24.4±0.2° and 26.1±0.2°.
6. A method for preparing the crystal form I according to any one of technical schemes 1-5, comprising:
   dissolving a compound of formula (I) in a single or mixed solvent, heating and cooling, and optionally, repeating the process of "heating and cooling" 1-2 times to give the crystal form I; wherein
   the single or mixed solvent is selected from one of or a mixture of two or more of methanol, ethanol, isopropanol, toluene, acetone, tetrahydrofuran, dichloromethane, dichloroethane, ethyl acetate, acetonitrile, methyl tert-butyl ether, 2-methyltetrahydrofuran, dimethyl sulfoxide and water; and preferably, the single or mixed solvent is selected from methanol, ethanol, ethyl acetate, acetonitrile, tetrahydrofuran, isopropanol, methyl *tert-butyl* ether, acetone, toluene, dichloromethane, isopropanol/water, and ethanol/water.
7. A pharmaceutical composition comprising the crystal form I according to any one of technical schemes 1-5 and one or more second therapeutically active agents.
8. A pharmaceutical formulation comprising the crystal form I according to any one of technical schemes 1-5, wherein the pharmaceutical formulation comprises one or more pharmaceutically acceptable carriers.
9. Use of the crystal form I according to any one of technical schemes 1-5 or the pharmaceutical composition according to technical scheme 7 or the pharmaceutical formulation according to technical scheme 8 in preparing a medicament for treating or preventing a related disease mediated by FGF/FGFR.
10. The use according to technical scheme 9, wherein the related disease is a related disease caused by changes in FGFR signaling pathway.
11. The use according to any one of technical schemes 9-10, wherein the related disease is a cancer.
12. The use according to any one of technical schemes 9-11, wherein the related disease is a solid tumor and a hematological tumor.
13. The use according to any one of technical schemes 9-12, wherein the related disease is selected from gallbladder carcinoma, cholangiocarcinoma, lung cancer, squamous cell carcinoma, bladder cancer, gastric cancer, ovarian cancer, peritoneal cancer, breast cancer, breast ductal carcinoma, head and neck cancer, endometrial cancer, corpus carcinoma, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, large intestine villous adenoma, melanoma, cytoma, sarcoma, myelodysplastic syndrome, and a combination thereof.

### BRIEF DESCRIPTION OF THE DRAWINGS

FIG. 1 is an X-ray powder diffraction (XRPD) pattern of the crystal form I of the compound of formula (I).

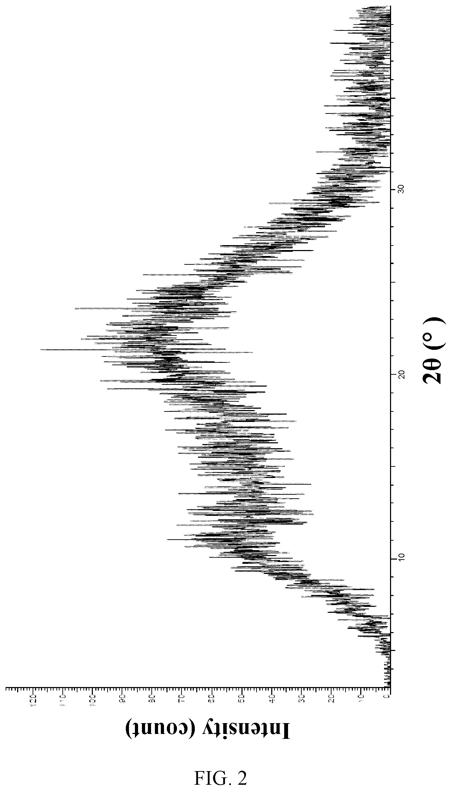

FIG. 2 is an XRPD pattern of an amorphous form sample of the compound (I).
FIG. 3 is a high-performance liquid chromatogram of related substances in the amorphous form on day 0.
FIG. 4 is a high-performance liquid chromatogram of related substances in the amorphous form after 10 days of storage at 105 °C.
FIG. 5 is a high-performance liquid chromatogram related substances in the amorphous form after 30 days of storage at 60 °C.

### EMBODIMENTS

The above description of the present invention is explained in further detail by the following description of specific embodiments, but it should not be construed that the scope of the present invention is limited to the following examples. All techniques realized based on the above description of the present invention shall fall within the scope of the present invention.

### Example 1. Preparation of compound of formula (I)

### Step 1: Synthesis of 1-tert-butyl 2-methyl(2S,4S)-4-(((benzyloxy)carbonyl)amino)pyrrolidine-1,2-dicarboxylate

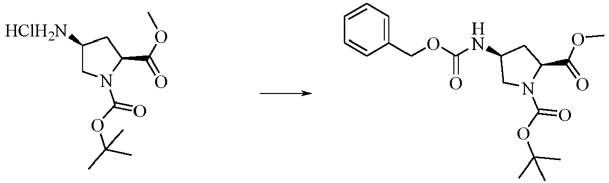

Starting material 1-(*tert*-butyl)2-methyl(2*S*,4*S*)-4-aminopyrrolidine-1,2-dicarboxylate hydrochloride (10.5 g, 36.8 mmol, 1.0 eq) was dissolved in dichloromethane (100 mL), and triethylamine (11.7 g, 115.8 mmol, 3.0 eq) was added. The resulting mixture was stirred and reacted at room temperature for 0.5 h and cooled to 0 °C in an ice bath. A solution of benzyl chloroformate (7.9 g, 46.3 mmol, 1.2 eq) in dichloromethane was added dropwise and slowly with a constant pressure dropping funnel. The resulting mixture was gradually warmed to room temperature and reacted overnight when the reaction was completed as monitored by TLC. A saturated sodium bicarbonate solution (100 mL) was added to the reaction solution, and the liquids were separated. The aqueous phase was extracted with dichloromethane (50 mL × 2). The organic phases were combined, washed with saturated brine, dried over anhydrous sodium sulfate, and filtered. The filtrate was concentrated, and the crude product was subjected to silica gel column chromatography (200-300 mesh silica gel, PE:EA = 10:1-3:1) to give 1-*tert*-butyl 2-methyl(2*S*,4*S*)-4-(((benzyloxy)carbonyl)amino)pyrrolidine-1,2-dicarboxylate as a colorless oil (3.6 g, 24.7% yield).

### Step 2: Synthesis of tert-butyl (2S,4S)-4-((((benzyloxy)carbonyl)amino)-2-(hydroxymethyl)pyrrolidine-1 -carboxylate

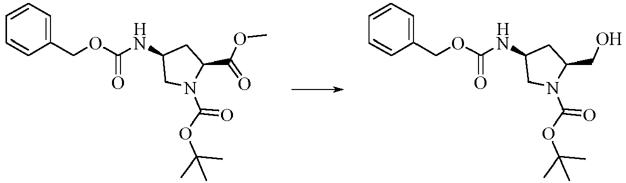

Lithium aluminum hydride (0.7 g, 19.0 mmol, 2.0 eq) was dissolved in anhydrous tetrahydrofuran (20 mL) at 0 °C. The resulting solution was stirred and reacted for half an hour, and a solution of *1-tert-butyl* 2-methyl(2*S*,4*S*)-4-(((benzyloxy)carbonyl)amino)pyrrolidine-1,2-dicarboxylate (3.6 g, 9.5 mmol, 1.0 eq) in tetrahydrofuran was added dropwise and slowly. The resulting mixture was gradually warmed to room temperature and reacted for 2 h when the reaction was completed as detected by TLC. The reaction solution was cooled to 0 °C, and water (0.7 mL) and a 10% sodium hydroxide solution (0.7 mL) were added dropwise and slowly to the reaction solution, followed by addition of water (2.1 mL). The reaction solution was stirred for half an hour and filtered. The filtrate was concentrated, and the crude product was subjected to silica gel column chromatography (200-300 mesh, PE:EA = 10:1 to 2:1) to give *tert*-butyl (2*S*,4*S*)-4-((((benzyloxy)carbonyl)amino)-2-(hydroxymethyl)pyrrolidine-1-carboxylate as a colorless oil (1.05 g, 30.0% yield).

### Step 3: Synthesis of tert-butyl (2S,4S)-4-amino-2-(hydroxymethyl)pyrrolidine-1-carboxylate

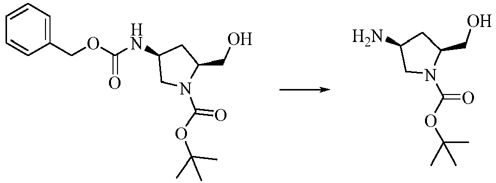

*tert-Butyl* (2*S*,4*S*)-4-((((benzyloxy)carbonyl)amino)-2-(hydroxymethyl) pyrrolidine-1-carboxylate (250 mg, 0.71 mmol, 1.0 eq) was dissolved in methanol (5.0 mL) and palladium on carbon (75.2 mg, 7.2 mmol, 0.01 eq) was added. The resulting mixture was purged with hydrogen 4 times, and stirred and reacted at room temperature overnight when the reaction was completed as monitored by TLC. The reaction solution was and filtered under vacuum through celite, and the filtrate was concentrated to give *tert*-butyl (2*S*,4*S*)-4-amino-2-(hydroxymethyl)pyrrolidine-1-carboxylate as a colorless transparent oil (123.0 mg, 80.2% yield).

### Step 4: Synthesis of tert-butyl (2S,4S)-4-((6-(2,6-dichloro-3,5-dimethoxyphenyl)quinazoline)-2-amino)-2-(hydroxymethyl)pyrrolidine-1-carboxylate

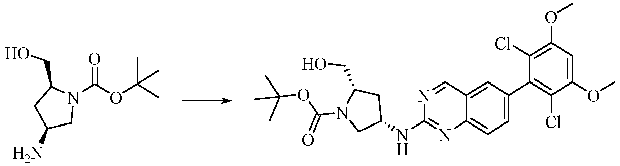

Starting materials tert-butyl (2*S*,4*S*)-4-amino-2-(hydroxymethyl)pyrrolidine-1-carboxylate(123.0 mg, 0.57 mmol, 1.0 eq) and 2-chloro-6-(2,6-dichloro-3,5-dimethoxyphenyl)quinazoline (231.5 mg, 0.63 mmol, 1.1 eq) were dissolved in *N-*methylpyrrolidone (4.0 mL), and *N*,*N*-diisopropylethylamine (147.3 mg, 1.14 mmol, 2.0 eq) was added. The resulting mixture was gradually warmed to 110 °C, stirred for 5 h, and the reaction was completed as monitored by TLC. The reaction solution was cooled to room temperature, and 20 mL of ice water was added to the reaction solution. The resulting mixture was stirred for 10 min and filtered. The filter cake was washed with a small amount of ice water, and then dissolved in dichloromethane (10 mL). The organic phases were dried over anhydrous sodium sulfate and filtered. The filtrate was concentrated, and the crude product was subjected to silica gel column chromatography (200-300 mesh, DCM:MeOH = 100:1 to 50:1) to give *tert*-butyl (2*S*,4*S*)-4-((6-(2,6-dichloro-3,5-dimethoxyphenyl)quinazolin-2-yl)amino)-2-(hydroxymethyl)pyrrolidine-1-carboxylate as a pale brown solid (113.0 mg, 36.1% yield).

### Step 5: Synthesis of ((2S,4S)-4-((6-(2,6-dichloro-3,5-dimethoxyphenyl)quinazoline)-2-amino)pyrrolidine)-2-methanol hydrochloride

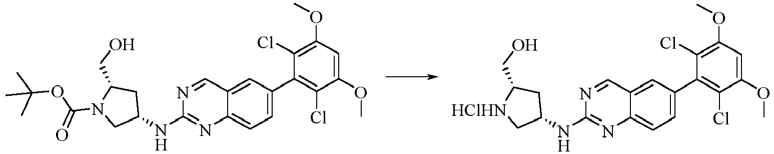

Starting material *tert*-butyl (2*S*,4*S*)-4-((6-(2,6-dichloro-3,5-dimethoxyphenyl)quinazolin-2-yl)amino)-2-(hydroxymethyl)pyrrolidine-1-carboxylate (113.0 mg, 0.21 mmol, 1.0 eq) was dissolved in ethanol (5.0 mL). The resulting mixture was cooled to 0 °C in an ice bath, and a solution of hydrogen chloride in ethanol (5.0 mL) was added. The resulting mixture was then gradually warmed to room temperature and reacted overnight when the reaction was completed as monitored by TLC. The reaction solution was directly concentrated to give ((2*S*,4*S*)-4-((6-(2,6-dichloro-3,5-dimethoxyphenyl)quinazoline)-2-amino)pyrrolidine)-2-methanol hydrochloride as a yellow solid (180.0 mg of crude product, yield: based on 100%).

### Step 6: Synthesis of 1-((2S,4S)-4-((6-(2,6-dichloro-3,5-dimethoxyphenyl)quinazolin-2-yl)amino)-2-(hydroxymethyl)pyrrolidin-1 -yl)prop-2-en-1 - one

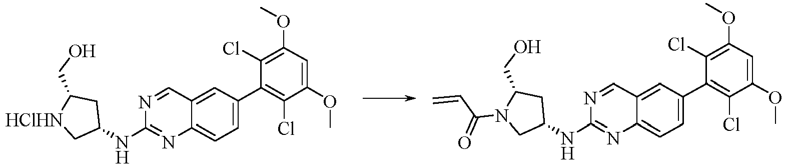

Starting material ((2*S*,4*S*)-4-((6-(2,6-dichloro-3,5-dimethoxyphenyl)quinazoline)-2-amino)pyrrolidine)-2-methanol hydrochloride (160.0 mg, 0.35 mmol, 1.0 eq) was dissolved in tetrahydrofuran (10.0 mL), and triethylamine (106.0 mg, 1.1 mmol, 3.0 eq) was added. The resulting mixture was stirred and reacted at room temperature for half an hour and cooled to 0 °C. Acryloyl chloride (38.0 mg, 0.42 mmol, 1.2 eq) was added dropwise and slowly. The resulting mixture was gradually warmed to room temperature and reacted for 1 h when the reaction was completed as monitored by TLC. A saturated sodium bicarbonate solution (15 mL) was added to the reaction solution, followed by addition of ethyl acetate (8 mL). The liquids were separated, and the aqueous phase was extracted with ethyl acetate (8 mL × 2). The organic phases were combined, washed with saturated brine, dried over anhydrous sodium sulfate, and filtered. The filtrate was concentrated, and the crude product was subjected to silica gel column chromatography (200-300 mesh silica gel, DCM:MeOH = 100:1 to 50:1) to give 1-((2*S*,4*S*)-4-((6-(2,6-dichloro-3,5-dimethoxyphenyl)quinazolin-2-yl)amino)-2-(hydroxymethyl)pyrrolidin-1 -yl)propyl-2-en-1 - one as a pale yellow solid (75.0 mg, 42.5% yield).

¹H NMR (400MHz, DMSO) δ (ppm): 9.18 (s, 1H), 7.89 (s, 1H), 7.80 (s, 1H), 7.55 (d, 1H), 7.52 (d, 1H), 7.00 (s, 1H), 6.60-6.71 (m, 1H), 6.16 (d, 1H), 5.67 (d, 1H), 5.09 (s, 1H), 4.55 (m, 1H), 4.12-4.20 (m, 2H), 4.03 (s, 6H), 3.97 (m, 1H), 3.74 (m, 1H), 3.52 (m, 1H), 1.98 (m, 2H).

Molecular formula: C₂₄H₂₄Cl₂N₄O₄; molecular weight: 503.39 LC-MS (Pos, m/z) = 505.40 [M+H⁺].

### Example 2. Preparation of crystal form I of compound of formula (I)

4.8 g of the compound of formula (I) was added to 20 mL of ethanol:water = 3:1, and the resulting mixture was heated to 80 °C. The heating was stopped after 3 h before the mixture was cooled to 40 °C, and a solid was precipitated. The mixture was heated to 80 °C again, and a large amount of solid was precipitated. The heating was stopped after 2.5 h. The mixture was then cooled to room temperature and filtered, and the filter cake was dried to give the crystal form I. The XRPD pattern is shown in FIG. 1. The X-ray powder diffraction pattern is shown in the figure below, and the main parameters are as follows:

| 2θ angle (°) | Intensity (%) |
|---|---|
| 5.2 | 66.9 |
| 10.5 | 93.1 |
| 11.5 | 13.8 |
| 12.9 | 10.3 |
| 13.9 | 100 |
| 16.0 | 51 |
| 16.4 | 39.3 |
| 18.9 | 13.1 |
| 20.9 | 25.5 |
| 22.4 | 12.4 |
| 24.4 | 36.6 |
| 26.1 | 37.2 |

### Example 3. Preparation of crystal form I of compound of formula (I)

300 mg of the compound of formula (I) was added into a reaction flask and heated to 70 °C, followed by addition of 2 mL of methanol. The resulting mixture was heated for half an hour before the heating was stopped. The mixture was cooled to room temperature, and the crystal form I was precipitated.

### Example 4. Preparation of crystal form I of compound of formula (I)

300 mg of the compound of formula (I) was added into a reaction flask, and 3 mL of ethyl acetate was added at 80 °C. The resulting mixture was heated for 1 h before the heating was stopped. The mixture was cooled to room temperature, and the crystal form I was precipitated.

### Example 5. Preparation of crystal form I of compound of formula (I)

300 mg of the compound of formula (I) was added into a reaction flask, and 3 mL of ethanol was added at 90 °C. The resulting mixture was heated for 1 h before the heating was stopped. The mixture was cooled to room temperature, and the crystal form I was precipitated.

### Example 6. Preparation of crystal form I of compound of formula (I)

300 mg of the compound of formula (I) was added into a reaction flask, and 4 mL of acetonitrile was added at 90 °C. The resulting mixture was heated for 1 h before the heating was stopped. The mixture was cooled to room temperature, and the crystal form I was precipitated.

### Example 7. Preparation of crystal form I of compound of formula (I)

300 mg of the compound of formula (I) was added into a reaction flask, and 3 mL of tetrahydrofuran was added at 80 °C. The resulting mixture was heated for half an hour before the heating was stopped. The mixture was cooled to room temperature and dried to give the crystal form I.

### Example 8. Preparation of crystal form I of compound of formula (I)

300 mg of the compound of formula (I) was added into a reaction flask, and 3 mL of isopropanol was added at 90 °C. The resulting mixture was heated for half an hour before the heating was stopped. The mixture was cooled to room temperature, and the crystal form I was precipitated.

### Example 9. Preparation of crystal form I of compound of formula (I)

300 mg of the compound of formula (I) was added into a reaction flask, and 10 mL of isopropanol:water = 4:6 was added at 90 °C. The resulting mixture was heated for half an hour before the heating was stopped. The mixture was cooled to room temperature, and the crystal form I was precipitated.

### Example 10. Preparation of crystal form I of compound of formula (I)

300 mg of the compound of formula (I) was added into a reaction flask, and 10 mL of methyl *tert*-butyl ether was added at 50 °C. The resulting mixture was heated for half an hour and the compound of formula (I) was not dissolved before the heating was stopped. The mixture was cooled to room temperature and filtered to give the crystal form I.

### Example 11. Preparation of crystal form I of compound of formula (I)

300 mg of the compound of formula (I) was added into a reaction flask, and 2 mL of acetone was added at 70 °C. The resulting mixture was heated for half an hour before the heating was stopped. The mixture was cooled to room temperature, and the crystal form I was precipitated.

### Example 12. Preparation of crystal form I of compound of formula (I)

300 mg of the compound of formula (I) was added into a reaction flask, and 3 mL of isopropanol was added at 100 °C. The resulting mixture was heated for half an hour before the heating was stopped. The mixture was cooled to room temperature, and the crystal form I was precipitated.

### Example 13. Preparation of crystal form I of compound of formula (I)

300 mg of the compound of formula (I) was added into a reaction flask, and 2 mL of dichloromethane was added at 40 °C. The resulting mixture was heated for half an hour before the heating was stopped. The mixture was cooled to room temperature and dried to give the crystal form I.

### Example 14. Preparation of crystal form I of compound of formula (I)

300 mg of the compound of formula (I) was added into a reaction flask, and 5 mL of ethanol:water = 8:2 was added at 90 °C. The resulting mixture was heated for half an hour before the heating was stopped. The mixture was cooled to room temperature, and the crystal form I was precipitated.

The crystal forms prepared in Examples 3-14 demonstrated similar XRPD patterns to FIG. 1. Thus, the crystal forms prepared in Examples 3-14 are all the crystal form I.

The above description is only for the purpose of illustrating preferred examples of the present invention, and is not intended to limit the scope of the present invention. Any modifications, equivalents, improvements and the like made without departing from the spirit and principle of the present invention should be included in the protection scope of the present invention.

### Experimental Example 1: Stability test of crystal form I of the present invention

Methodology: Proper amounts of crystal form I samples were placed into weighing bottles, and the bottles were let stand open under the conditions of 60 °C, RH 92.5%, RH 75% and illumination (≥ 4500±500 Lux), respectively. Samples were taken on days 0, 4 and 10, and investigated for changes in properties and purity.

### Results:

**Table 1: Test results under influencing factors**

| Conditions | Appearance | Purity % |
|---|---|---|
| Day 0 | Off-white powder | 99.6 |
| 60 °C, Day 4 | Off-white powder | 99.6 |
| 60 °C, Day 10 | Off-white powder | 99.6 |
| 75% RH, Day 4 | Off-white powder | 99.6 |
| 75% RH, Day 10 | Off-white powder | 99.6 |
| 92.5% RH, Day 4 | Off-white powder | 99.6 |
| 92.5% RH, Day 10 | Off-white powder | 99.6 |
| Illumination, Day 4 | Off-white powder | 99.6 |
| Illumination, Day 10 | Pale yellow powder | 99.5 |

Conclusion: After 10 days in various influencing factor conditions, the appearance and purity of the crystal form I of the present invention did not change significantly, demonstrating good stability.

### Experimental Example 2: Stability test of amorphous form and crystal form I

Methodology: Proper amounts of crystal form I samples were placed into weighing bottles. The bottles were let stand at 105 °C (samples were taken on days 3, 5 and 10) and at 60 °C (samples were taken on days 10, 20 and 30). The taken samples were investigated for changes in appearance, related substances and form.

Proper amounts of amorphous form samples of the compound (I) were placed into weighing bottles. The bottles were let stand at 105 °C (samples were taken on days 3, 5 and 10) and at 60 °C (samples were taken on days 10, 20 and 30). The taken samples were investigated for changes in appearance, related substances.

### The results are as follows:

**Table 2: Related substances in stability test of crystal form I**

| Conditions | Related substances (%) | Destructive impurity (%) |
|---|---|---|
| Day 0 | 0.64 | None |
| 105 °C, Day 3 | 0.62 | Undetectable |
| 105 °C, Day 5 | 0.60 | Undetectable |
| 105 °C, Day 10 | 0.63 | Undetectable |
| 60 °C, Day 10 | 0.64 | Undetectable |
| 60 °C, Day 20 | 0.64 | Undetectable |
| 60 °C, Day 30 | 0.68 | Undetectable |

**Table 3: Appearance and XRPD in stability test of crystal form I**

| Conditions | Appearance | Form |
|---|---|---|
| Day 0 | Off-white powder | Crystal form I |
| 105 °C, Day 3 | Off-white powder | Crystal form I |
| 105 °C, Day 5 | Off-white powder | Crystal form I |
| 105 °C, Day 10 | Off-white powder | Crystal form I |
| 60 °C, Day 10 | Off-white powder | Crystal form I |
| 60 °C, Day 20 | Off-white powder | Crystal form I |
| 60 °C, Day 30 | Off-white powder | Crystal form I |

**Table 4: Related substances and appearance in stability test of amorphous form**

| Conditions | Related substances (%) | Appearance | Destructive impurity (%) |
|---|---|---|---|
| Day 0 | 0.62 | Pale yellow powder | None |
| 105 °C, Day 3 | 1.3 | Pale yellow powder | 0.27/0.41 |
| 105 °C, Day 5 | 1.6 | Pale yellow powder | 0.02/0.23/0.63 |
| 105 °C, Day 10 | 2.5 | Pale yellow powder | 0.04/0.02/0.03/0.21/ 1.29/0.02/0.02 |
| 60 °C, Day 10 | 0.73 | Pale yellow powder | 0.06 |
| 60 °C, Day 20 | 0.72 | Pale yellow powder | 0.06/0.01 |
| 60 °C, Day 30 | 0.75 | Pale yellow powder | 0.05/0.02 |

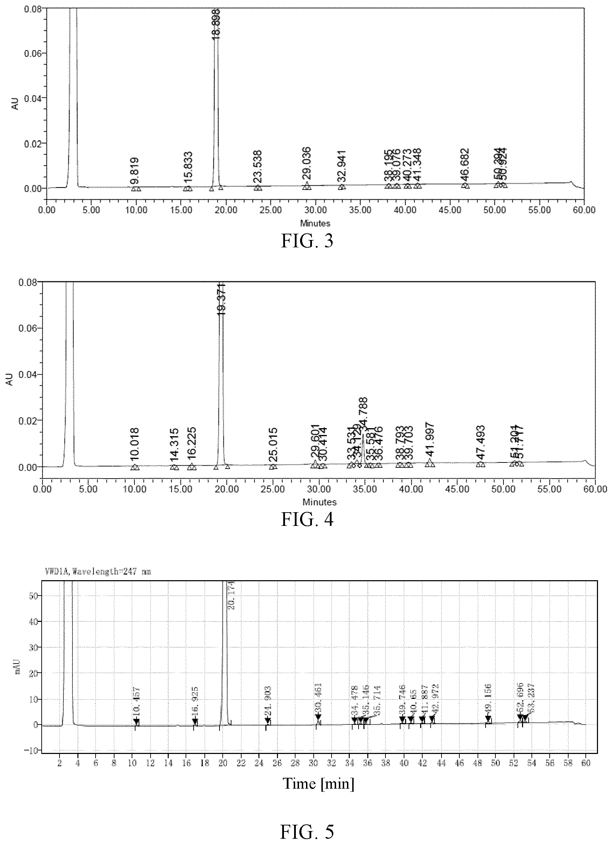

Conclusion: After storage at 105 °C for 10 days and at 60 °C for 30 days, the appearance, related substances and form of the crystal form I of the present invention did not change significantly, demonstrating good stability. The HPLC analysis of the amorphous form on day 0 is shown in FIG. 3. After the amorphous form was stored at 105 °C for 10 days, 7 destructive impurities were detected, with retention times of 14.315 min, 25.015 min, 30.414 min, 34.129 min, 34.788 min, 35.581 min and 36.476 min, respectively. The HPLC analysis is shown in FIG. 4. After the amorphous form was stored at 60 °C for 30 days, 2 destructive impurities were detected, with retention times of 35.146 min and 35.714 min, respectively. The HPLC analysis is shown in FIG. 5.

### Experimental Example 3: Cell viability assay

SNU-16 is a gastric cancer cell with FGFR abnormalities.
Test samples: Amorphous form and crystal form I of compound (I)
Instrument: Espire multi-functional microplate reader

### Methodology:

SNU-16 cells were seeded in a 96-well plate for adherent culture overnight, and then compounds at different concentrations (12 dose groups, serial 3-fold dilution with DMSO) were added to give final concentrations of 0.17-30000 nM, wherein the final DMSO content was 5‰. Negative control wells contained a 5‰ DMSO medium. The plate was incubated at 37 °C, 5% CO₂ and 95% humidity for 72 h for later test. 30 µL of Cell titer-Glo reagent was added to each well, and after the plate was incubated at room temperature for 30 min, the final data were read by Espire.

The test results are shown in Table 5 below.

**Table 5: Inhibitory activity on cells (IC₅₀)**

| Compound | Inhibitory activity (nM) on SNU-16 cells |
|---|---|
| Amorphous form | 11 |
| Crystal form I | 9.5 |

It can be seen from the above table that the crystal form I of the present invention had good inhibitory activity on SNU-16 cells with FGFR abnormalities, indicating that the compound of the present invention can be used for treating cancers mediated by FGF/FGFR abnormalities, such as gastric cancer.

## Claims

1. A crystal form I of compound 1-((2*S*,4*S*)-4-((6-(2,6-dichloro-3,5-dimethoxyphenyl)quinazolin-2-yl)amino)-2-(hydroxymethyl)pyrrolidin-1 -yl)prop-2-en-1 - one of formula (I), wherein the crystal form I comprises characteristic peaks in an X-ray powder diffraction pattern using Cu-Kα radiation at 2θ angles of 5.2±0.2°, 10.5±0.2°, 13.9±0.2°, 16.0±0.2°, 20.9±0.2°, 24.4±0.2° and 26.1±0.2°,

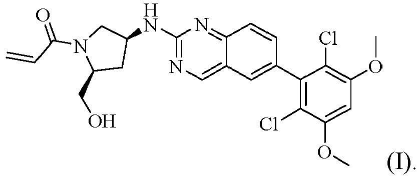

2. The crystal form I according to claim 1, further comprising characteristic peaks in an X-ray powder diffraction pattern using Cu-Kα radiation at 2θ angles of 11.5±0.2°, 12.9±0.2° and 22.4±0.2°.

3. The crystal form I according to claim 2, having an X-ray powder diffraction pattern using Cu-Kα radiation as substantially shown in FIG. 1.

4. A method for preparing the crystal form I according to claim 1, comprising:
dissolving a compound of formula (I) in a single or mixed solvent, heating and cooling, and optionally, repeating the process of "heating and cooling" 1-2 times to give the crystal form I; wherein
the single or mixed solvent is selected from one of or a mixture of two or more of methanol, ethanol, isopropanol, toluene, acetone, tetrahydrofuran, dichloromethane, dichloroethane, ethyl acetate, acetonitrile, methyl *tert*-butyl ether, 2-methyltetrahydrofuran, dimethyl sulfoxide and water; and preferably, the single or mixed solvent is selected from methanol, ethanol, ethyl acetate, acetonitrile, tetrahydrofuran, isopropanol, methyl *tert*-butyl ether, acetone, toluene, dichloromethane, isopropanol/water, and ethanol/water.

5. A pharmaceutical composition comprising the crystal form I according to any one of claims 1-3 and one or more second therapeutically active agents.

6. A pharmaceutical formulation comprising the crystal form I according to any one of claims 1-3, wherein the pharmaceutical formulation comprises one or more pharmaceutically acceptable carriers.

7. Use of the crystal form I according to any one of claims 1-3 or the pharmaceutical composition according to claim 5 or the pharmaceutical formulation according to claim 6 in preparing a medicament for treating or preventing a related disease mediated by FGF/FGFR.

8. The use according to claim 7, wherein the related disease mediated by FGF/FGFR is a related disease caused by changes in FGFR signaling pathway, and preferably a cancer; more preferably, the cancer is a solid tumor or a hematological tumor; still more preferably, the cancer includes gallbladder carcinoma, cholangiocarcinoma, lung cancer, squamous cell carcinoma, bladder cancer, gastric cancer, ovarian cancer, peritoneal cancer, breast cancer, breast ductal carcinoma, head and neck cancer, endometrial cancer, corpus carcinoma, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, large intestine villous adenoma, melanoma, cytoma, sarcoma and myelodysplastic syndrome.
